(19) Europäisches Patentamt
European Patent Office
Office européen des brevets

(11) **EP 3 011 973 B1**

(12) **EUROPEAN PATENT SPECIFICATION**

(45) Date of publication and mention
of the grant of the patent:
**16.01.2019 Bulletin 2019/03**

(21) Application number: **14813986.8**

(22) Date of filing: **13.06.2014**

(51) Int Cl.:
**A61K 9/36** *(2006.01)*    **A61K 47/38** *(2006.01)*

(86) International application number:
**PCT/JP2014/065740**

(87) International publication number:
**WO 2014/203819 (24.12.2014 Gazette 2014/52)**

(54) **COATING AGENT CONTAINING HYDROXYALKYL CELLULOSE**

BESCHICHTUNGSMITTEL MIT HYDROXYALKYLCELLULOSE

AGENT D'ENROBAGE CONTENANT UNE HYDROXYALKYLCELLULOSE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **17.06.2013 JP 2013126671**

(43) Date of publication of application:
**27.04.2016 Bulletin 2016/17**

(73) Proprietor: **Nippon Soda Co., Ltd.**
**Tokyo 100-8165 (JP)**

(72) Inventors:
• **UMEZAWA Tadashi**
**Joetsu-shi**
**Niigata 949-2392 (JP)**
• **SHIMOTORI Takeshi**
**Joetsu-shi**
**Niigata 949-2392 (JP)**
• **TSUE Shinichiro**
**Joetsu-shi**
**Niigata 949-2392 (JP)**

(74) Representative: **Wills, Andrew Jonathan et al**
**Mewburn Ellis LLP**
**City Tower**
**40 Basinghall Street**
**London EC2V 5DE (GB)**

(56) References cited:
EP-A1- 1 588 708       EP-A1- 1 967 183
WO-A1-2011/027728   JP-A- 2001 342 185
US-A- 4 258 179        US-A1- 2006 177 509

• RJ HARWOOD: "HYDROXYPROPYL CELLULOSE" In: Royal Pharmaceutical Society of Great Britain: "Handbook of Pharmaceutical Excipients", 2006, Pharmaceutical Press; The American Pharmacists Association, UK/USA, XP002765577, ISBN: 0-85369-618-7 pages 336-340, * the whole document *
• 'Hydroxypropyl cellulose, with respect to technical information' NISSO HPC 31 July 2012, XP055300459 Retrieved from the Internet: <URL:https://web.archive.org/web/20120731053540/http://www.nissoexcipients.com/JP/Default.aspx>
• KYOSUKE TSUDA ET AL. IYAKUHIN KAIHATSU KISO KOZA XI YAKUZAI SEIZOHO 1971, pages 222 - 233, XP008182669
• 'Japan Pharmaceutical Excipients Council' HANDBOOK OF PHARMACEUTICAL EXCIPIENTS 28 February 2007, page 690, 700, XP008153603

**Description**

[Technical Field]

The present invention relates to a coating agent containing a hydroxyalkyl cellulose. More specifically, the present invention relates to a coating agent suitable for obtaining a coating film of solid formulations for medicine, agricultural chemicals or food.

Priority is claimed on Japanese Patent Application No. 2013-126671, filed June 17, 2013.

[Background Art]

Hydroxypropyl celluloses are non-ionic polymers in which a hydroxyl group in a glucose ($C_6H_{10}O_5$) unit serving as a structural unit of cellulose is etherified with a hydroxypropyl group. With respect to the hydroxypropyl celluloses, those in which the amount of hydroxypropyl groups is from 53.4 to 77.5% by mass, those in which the amount of hydroxypropoxyl groups is from 40 to 50% by mass, and those in which the amount of hydroxypropoxyl groups is from 5 to 16% by mass are known. In general, the hydroxypropyl celluloses in which the amount of hydroxypropyl groups is from 5 to 16% by mass are called as low-substituted hydroxypropyl celluloses (refer to Patent Document 1 or the like).

It has been known that a hydroxyalkyl cellulose is used as a coating film of solid formulations. However, this coating film of hydroxyalkyl cellulose may cause aggregation which is called blocking.

Thus, Patent Document 2 has proposed a hydroxypropyl cellulose having an average substitution mole number of 2 to 3, in which the ratio of both unsubstituted products and highly substituted products having a hydroxypropyl group substitution mole number of 4 or more is low, whereby anti-blocking can be achieved under the environment of 25°C and 75% RH.

In addition, in Patent Document 3, a coating composition containing a low-substituted hydroxypropyl cellulose, talc, propylene glycol and polyethylene glycol has been proposed, whereby it has been shown that a tablet with no adhesion property can be provided.

In addition, Patent Document 4 discloses that the use of a coating agent containing a hydroxyalkyl cellulose in which an amount of a hydroxyalkyl group is within a range of 40% to 50% by mass makes it possible to provide a tablet in which the occurrence of blocking due to stickiness is prevented even under a high temperature and high humidity severe environment such as around 50°C and 90% RH.

[Citation List]

[Patent Documents]

[0008]

　　[Patent Document 1] Japanese Laid-open Patent Application No. 2001-31701
　　[Patent Document 2] Japanese Unexamined Patent Application, First Publication No. Hei 9-202801
　　[Patent Document 3] Japanese Laid-open Patent Application No. 2007-1873
　　[Patent Document 4] WO 2011-027728
　　[Patent Document 5] Japanese Laid-open Patent Application No. 2002-207030

US 2006/0177509 A1 discloses a controlled release composition showing release of a proton pump inhibitor controlled in two or more steps at different release rates. The composition contains a release-controlled part A capable of controlling release at a predetermined rate and a release controlled part B capable of controlling release at a lower predetermined rate than the rate of part A.

US 4258179 A discloses a coating agent for solid medicaments containing a hydrogel-like substance of a water-insoluble hydroxypropyl cellulose having 5-16% by weight of a hydroxypropoxy group or the dry powder thereof.

EP 1967183 A1 discloses a controlled release solid preparation containing (1) an antacid, (2) an immediate-release part containing a compound unstable to acid and a basic substance, and (3) a sustained-release part containing a compound unstable to acid and a pH-independent material in combination.

EP 1588708 A1 discloses a method of producing a preparation coated with pioglitazone hydrochloride, useful as a therapeutic agent for diabetes and the like.

US 2012/0157674 A1 discloses a coating agent containing a hydroxyalkyl cellulose in which a content of hydroxyalkyl groups within the hydroxyalkyl cellulose is within a range of 40 to 50% by mass, and a solid preparation coated with the coating agent.

[Summary of Invention]

[Technical Problem]

The present invention has an object of providing a coated tablet having a high hardness with almost no loss due to friction and having a lengthened disintegration time.

[Solution to Problem]

The invention is set out in claim 1.
The present disclosure also includes the following aspects (1) to (6), which do not correspond to the invention:

(1) A coating agent containing 1% to 7% by mass, relative to a total mass of the coating agent, of a hydroxyalkyl cellulose having hydroxyalkyl groups in an amount of more than 50% to 60% by mass, relative to the total mass of the hydroxyalkyl cellulose.
(2) The coating agent according to the above aspect (1) which is used to coat a tablet.
(3) The coating agent according to the above aspect (1) or (2), wherein the hydroxyalkyl cellulose has a viscosity of 3.0 to 5.9 mPa•s, when the hydroxyalkyl cellulose is made to be 2% by mass of an aqueous solution at 20°C.
(4) The coating agent according to any one of the above aspects (1) to (3), wherein the hydroxyalkyl group is a hydroxypropyl group.
(5) A solid formulation containing a coating formed with the coating agent described in any one of the above aspects (1) to (4).
(6) The solid formulation according to the above aspect (5), wherein the coating ratio is 2% to 6% by mass.

[Advantageous Effects of Invention]

It is possible to obtain a solid formulation having a high hardness with almost no loss due to friction and having a long disintegration time by spraying the coating agent according to the present disclosure onto a plain tablet and drying the coated tablet.

[Brief Description of Drawings]

FIG. 1 is a drawing showing a coated tablet prepared in Example 1.
FIG. 2 is a drawing showing a detailed surface of the coated tablet prepared in Example 1.
FIG. 3 is a drawing showing a coated tablet prepared in Comparative Example 2.
FIG. 4 is a drawing showing a detailed surface of the coated tablet prepared in Comparative Example 2.

[Description of Embodiments]

A coating agent of the present disclosure contains a hydroxyalkyl cellulose.
The hydroxyalkyl cellulose can be obtained, for example, by reacting sodium hydroxide with cellulose serving as a raw material to produce an alkali cellulose, and then allowing a substitution reaction between the alkali cellulose and an alkylene oxide to proceed.
Hydroxypropyl cellulose can be obtained through the substitution reaction by using propylene oxide.
Some or all of the -OH groups in the glucose ring unit of cellulose are substituted with $-O-(RO)_m-H$ groups by the substitution reaction. In the formula, R represents a divalent hydrocarbon group. R is preferably $-CH_2-CH(CH_3)$ or $-CH_2-CH_2$, and more preferably $-CH_2-CH(CH_3)$. m is a natural number of 1 or more.
After the substitution reaction, sodium hydroxide can be neutralized by adding an acid such as acetic acid or hydrochloric acid to the reaction solution, followed by purification.
The hydroxypropyl cellulose included in the coating agent according to the present invention has a hydroxypropyl group in an amount of more than 50% to 60% by mass, preferably in an amount of 51% to 58% by mass. When the amount of the hydroxypropyl group is within the range, the stickiness of the coating film is reduced, the occurrence of lack in hardness of the coating film is limited, and the occurrence of blocking is limited. It should be noted that the amount of hydroxypropyl group can be determined in accordance with the method described in USP24 (US Pharmacopeia) or the method described in Patent Document 5.
The amount of the hydroxypropyl cellulose, relative to the total mass of the coating agent according to the present invention, is 3% to 6% by mass. With this range, a solid formulation particularly suitable for an orally-disintegrating tablet in terms of hardness and disintegration time can be obtained.

The hydroxypropyl cellulose used in the present invention exhibits a viscosity, when the hydroxypropyl cellulose is made to be 2% by mass of an aqueous solution at 20°C, of 3.0 mPa•s to 5.9 mPa•s, and more preferably of 4.7 mPa•s to 5.9 mPa•s. The viscosity is an index indicating the degree of polymerization of hydroxypropyl cellulose. With this viscosity, the workability when obtaining the tablet is improved.

The coating agent is obtainable by dissolving or dispersing the aforementioned hydroxypropyl cellulose in a solvent.

Water, or an organic solvent, such as acetone, ethanol or isopropyl alcohol, is usually used as the solvent, and water is preferably used in view of its environmental safety, residual solvent safety, or the like.

It is preferable that an amount of the solvent, relative to the total mass of the coating agent according to the present invention, be 80% to 98% by mass, more preferably 85% to 97% by mass, and even more preferably 90% to 97% by mass.

In the coating agent, a known compounding agent usually used as a coating agent for tablets may be incorporated, in addition to the aforementioned hydroxypropyl cellulose. Examples thereof include powders such as talc, titanium oxide, ferric oxide yellow, iron sesquioxide, legal pigments, light anhydrous silicic acid and hydrous silicon dioxide; lubricants (plasticizers), such as polyethylene glycol, polypropylene glycol, triethyl citrate, glycerol mono-, di- or triacetate, 1,2-propylene glycol, castor oil, dibutyl sebacate, diethyl phthalate, polyethylene glycol methyl ether, phospholipid, and lecithin; adhesion promoters such as sucrose, polyvinylpyrrolidone, dextrose, sorbitol, mannitol, sucrose, polyvinylpyrrolidone, lactose, starch, sodium starch glycolate, ethyl cellulose and maltodextrins; and film forming agents such as cellulose acetate phthalate, microcrystalline cellulose, methyl cellulose, hydroxypropyl methylcellulose, alginates, gum arabic, carboxymethyl cellulose, hydroxyethyl cellulose and methyl cellulose.

Among these, lubricants (plasticizers) are preferably formulated. It is preferable that the amount of a lubricant, relative to the mass of the hydroxyalkyl cellulose, be approximately no less than 5% by mass and no more than 15% by mass, and more preferably no less than 7% by mass and no more than 12% by mass. In the case where the amount of the lubricant is within the above range, a solid formulation having a disintegration time and hardness, particularly suitable as an orally-disintegrating tablet can be obtained.

Plain tablets that are coated with the coating agent are prepared by the usual production method. For example, a plain tablet having a suitable size can be obtained by mixing and kneading drugs and excipients, binders, disintegrants, lubricants or the like with a small amount of water, organic solvents or the like, followed by the steps of granulation, drying, particle size control and tableting.

The amount of the coating agent to be coated on a plain tablet (coating ratio) is 4% to 5% by mass. The coating ratio is determined in accordance with the following formula. With this coating ratio, a tablet having a high hardness and a disintegration time, particularly suitable as an orally-disintegrating tablet, can be obtained.

$$\text{Coating ratio} \atop \text{(\% by mass)} = \frac{\text{Mass of solid preparation after coating} - \text{Mass of plane tablet}}{\text{Mass of plane tablet} + \text{Mass of solid content of coating agent used for coating}} \times 100$$

The thickness of a dried coating film is 45 μm to 65 μm.

The coating may usually be carried out using a sugar coating pan or a porous coating machine, usually at room temperature, or while heating at 20°C to 200°C in some cases. In the present invention, it is preferable that coating be performed by a spray process. It is preferable that the spray coating be performed by supplying air at 50°C to 70°C. It is preferable that an air flow rate when spray coating be 0.4 $m^3$/min to 0.6 $m^3$/min. It is preferable that a static pressure (gauge pressure) when spray coating be -5 Pa to -15 Pa. It is preferable that a spray pressure (gauge pressure) when spray coating be 0.5 MPa to 1.5 MPa. It is preferable that a tablet load amount when spray coating be 0.31 $g/cm^3$ to 0.54 $g/cm^3$ (brime amount). It is preferable that a liquid flow rate when spray coating be 3 mL/min to 5 mL/min. It is preferable that the spray liquid amount when spray coating be 300 g to 560 g (the solid content of which be 3% to 8% by mass).

The form of the solid formulation to be obtained is coated tablets. Moreover, the solid formulation obtained according to the present invention may be prepared in a sugar-coated tablet or the like. Furthermore, if the gloss is required, wax coating may be conducted with carnauba wax or the like in accordance with the conventional methods.

[Examples]

Next, the present invention is described in more detail, based on a series of examples. However, the present invention is in no way limited by these examples.

Example 1

Preparation of plain tablet

70 parts by mass of lactose for direct compression, 30 parts by mass of cornstarch, and 0.5 parts by mass of magnesium stearate were mixed, and then tableted at a tableting pressure of 10 kN in a tablet size of 8 mmφ - R tablet (200 mg/T) using a tableting machine manufactured by KIKUSUI SEISAKUSHO LTD. (VELA5) to obtain a plain tablet.

Production of hydroxypropyl cellulose (A)

176 g of ground pulp was placed into a reactor equipped with a stirrer, and then 68.2 g of a 20% NaOH aqueous solution was added thereto, followed by the addition of 602 g of toluene. The resulting mixture was stirred for 30 minutes and the temperature inside the reactor was adjusted to 30°C. A mercerization reaction was conducted for 1 hour by applying pressure with nitrogen while stirring inside the reactor.
After releasing the pressure, propylene oxide was added to the resultant. The amount of the added propylene oxide, as a molar ratio relative to the pulp, was 4.92.
Next, the temperature inside the reactor was increased to about 80°C. An etherification reaction was carried out by maintaining the temperature at 80°C for about 1 hour while stirring the resultant. Then, the temperature inside the reactor was lowered to 45°C or less. After 1 hour from the start of the cooling, the temperature was raised to 85°C and was maintained at 85°C for 1.5 hours. Then, the temperature inside the reactor was lowered to 40°C or less.
The product was washed out from the reactor with boiling water. The product obtained by washing was placed in a flask, and toluene was removed by distillation. After distillation, the resulting solution was allowed to stand. A gel was precipitated. The supernatant was decanted.
Boiling water was poured onto the gel, and the resultant was stirred for 10 minutes and was then allowed to stand again. A gel was precipitated. The supernatant was decanted. Boiling water was poured onto the gel. 60% acetic acid was then added thereto every ten minutes while stirring the mixture at about 85°C until the pH reached 4.9 or less. The temperature of the resultant was then adjusted to 90°C, and a predetermined amount of viscosity modifier was added thereto while stirring the mixture. The resulting mixture was stirred for 14 hours at 90°C.
A 20% NaOH aqueous solution was then added thereto every ten minutes while stirring the mixture at about 85°C until the pH reached 7.5. The resulting solution was then allowed to stand. A gel was precipitated. The supernatant was decanted. Boiling water was poured onto the gel. The resultant was stirred for 10 minutes and was then allowed to stand again. A gel was precipitated. The supernatant was decanted.
The gel was collected from the flask and cast on a flat plate made of fluorine resin. The resultant was vacuum dried at 70°C to obtain a hydroxypropyl cellulose (A) in which the amount of a hydroxypropyl group was 53.0% by mass.
The viscosity of a 2% aqueous solution of the obtained hydroxyalkyl cellulose (A) at 20°C was 4.88 mPa•s. Note that the viscosity of the 2% aqueous solution was measured at 20°C using a digital viscometer/B-type viscometer (DV-II + Pro, manufactured by Brookfield Engineering Laboratories) at 60 rpm.

Preparation of coated tablet

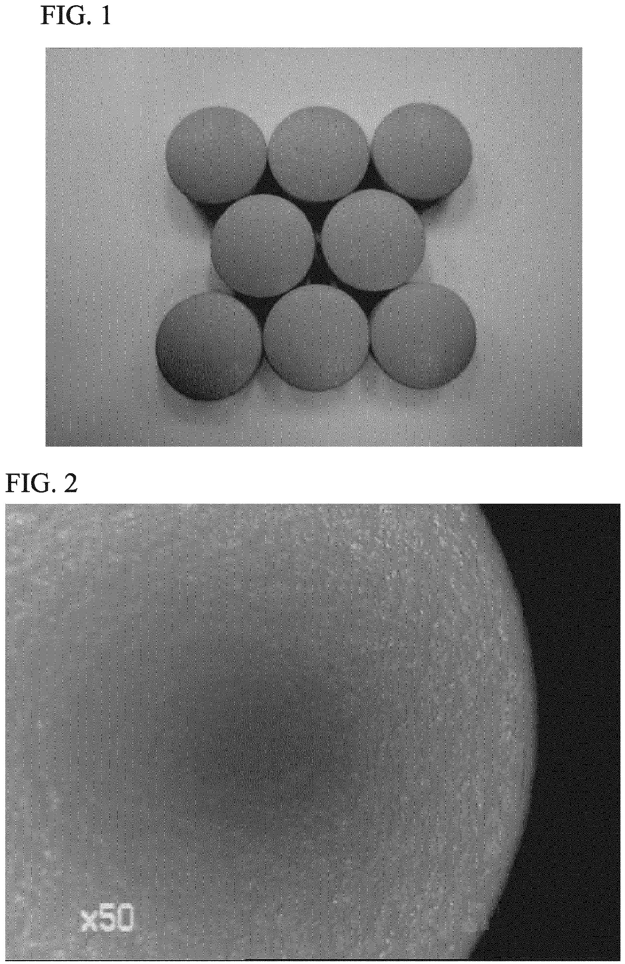

5 parts by mass of hydroxypropyl cellulose (A), 0.5 parts by mass of polyethylene glycol (PEG 6000), 0.01 parts by mass of a coloring agent (yellow No. 5), and 94.49 parts by mass of distilled water were mixed to prepare a spray liquid.
The spray liquid was sprayed onto the above-mentioned plain tablet using a coating apparatus (Hi-Coater LABO, pan size of 20 type) manufactured by FREUND CORPORATION at an air supply temperature of 60 °C, at an air flow rate of 0.5 $m^3$/min, at a static pressure of -10 Pa, at a spray pressure of 0.1 MPa, at a pan rotation speed of 20 rpm, at a tablet load amount of 300 g, at a liquid flow rate of 3 mL/min to 4 mL/min, and at a spraying liquid amount of 300 g (in which a HPC solid content was 15 g), to obtain a coated tablet according to the present invention. The microscopic appearance of the coated tablet according to the present invention is shown in Fig. 1 and Fig. 2.
The hardness, thickness, mass, disintegration time, and friability of the plain tablet and the coated tablet according to the present invention, and the coating film thickness were measured to calculate a coating ratio. The results are shown in Table 1.
The tablet hardness and the tablet thickness are mean values of 10 tablets measured using a load cell type portable tablet hardness tester (manufactured by OKADA SEIKO CO., LTD., Type PC-30).
The tablet mass is a mean value of 10 tablets measured using an electron scale.
The friability is a mean value of 30 tablets measured using a tablet friability tester (manufactured by Toyama Sangyo Co., Ltd., Type TFT-1200) (25 rpm, 100 rotations).

The disintegration time is a mean value of 6 tablets measured using a disintegration tester (manufactured by Toyama Sangyo Co., Ltd., Type NT-2) with distilled water at 37°C.

The coating film thickness is a mean value of 10 tablets calculated in accordance with the following formula.

$$\text{Coating film thickness} = \frac{\text{Tablet thickness of coated tablet} - \text{Tablet thickness of plane tablet}}{2}$$

Comparative Example 1

A hydroxypropyl cellulose (B), in which the amount of a hydroxypropyl group was 45.5% by mass, and of which the viscosity, when the hydroxypropyl cellulose (B) was made to be 2% by mass of an aqueous solution at 20°C, was 4.08 mPa · s, was obtained in a similar manner to that of Example 1, except that the additional amount of propylene oxide, as a molar ratio relative to pulp, was 3.68.

A coated tablet was prepared in a similar manner to that of Example 1, except that the hydroxypropyl cellulose (B) was used instead of the hydroxyproply cellulose (A) used in Example 1. The hardness, thickness, mass, disintegration time, friability, and coating film thickness of the coated tabled were measured, and the coating ratio was calculated in a similar manner to that of Example 1. The results are shown in Table 1.

Comparative Example 2

A hydroxypropyl cellulose (C), in which an amount of a hydroxypropyl group was 63.8% by mass, and of which the viscosity, when the hydroxypropyl cellulose (C) was made to be a 2% by mass of an aqueous solution at 20°C, was 4.54 mPa · s, was obtained in a similar manner to that of Example 1, except that an additional amount of propylene oxide, as a molar ratio relative to pulp, was 7.35.

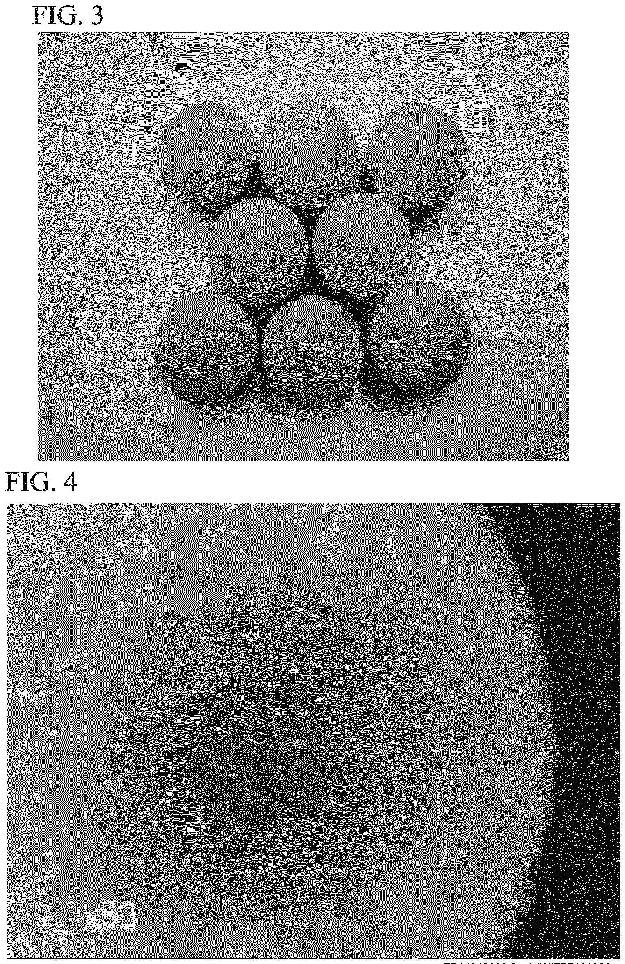

A coated tablet was prepared in a similar manner to that of Example 1, except that the hydroxypropyl cellulose (C) was used instead of the hydroxyproply cellulose (A) used in Example 1. The microscopic appearance of the coated tablet is shown in Fig.3 and Fig. 4. The hardness, thickness, mass, disintegration time, friability, and coating film thickness of the coated tabled were measured, and the coating ratio was calculated. The results are shown in Table 1.

Table 1

|  |  | Plane tablet | Comparative Example 1 | Example 1 | Comparative Example 2 |
|---|---|---|---|---|---|
| PO group | % by mass |  | 45.5 | 53.0 | 63.8 |
| Viscosity | mPa•s |  | 4.08 | 4.88 | 4.54 |
| Coating film tickness | μm |  | 41.0 | 55.2 | 72.0 |
| Coating ratio | % by mass |  | 3.77 | 4.56 | 4.23 |
|  |  |  |  |  |  |
| Tablet hardness | kgf | 8.95 | 8.85 | 9.18 | 8.87 |
| Tablet thickness | mm | 3.613 | 3.695 | 3.723 | 3.757 |
| Tablet mass | g (50 tablets) | 9820 | 10.190 | 10.268 | 10.236 |
| Tablet disintegration time | sec | 128 | 217 | 249 | 220 |
| Tablet friability | % by mass | 0.20 | 0.00 | 0.00 | 0.00 |

These results indicate an increase in the coating film thickness and the tablet thickness with an increase in the amount of the hydroxypropyl group. In contrast, although the coating ratio, the tablet hardness, and the tablet disintegration time increased with an increase in the amount of the hydroxypropyl group from Comparative Example 1 to Example 1, these values decreased with an increase in the amount of the hydroxypropyl group from Example 1 to Comparative Example 2. Although the tablet hardness of the coated tablet prepared in Example 1 increased relative to that of the plain tablet, that of coated tablets prepared in Comparative Examples 1 and 2 did not increase relative to that of the plain tablet. In view of the above results, it was confirmed that the coated tablet prepared in Example 1 was excellent in terms of the tablet hardness and the disintegration time compared to the coated tablets prepared in Comparative Examples 1 and 2.

In addition, the surface of the coated tablet prepared in Example 1 was smooth as shown in Fig. 1 and Fig. 2. In contrast, the surface of the coated tablet prepared in Comparative Example 2 was rough and the coating film was partially removed, as shown in Fig. 3 and Fig. 4, and thereby the coated tablet lacked uniformity of biological effects due to the promoted drug dissolution from the removed portion and the like.

[Industrial Applicability]

A solid formulation having a high hardness with suppressed loss due to friction and having a long disintegration time can be obtained by spraying the coating agent according to the present invention onto a plain tablet and drying the coated tablet.

## Claims

1. A solid formulation comprising: a plain tablet; and a coating on the plain tablet, wherein the coating has a thickness of from 45 $\mu$m to 65 $\mu$m and is formed with a coating agent comprising 3% to 6% by mass, relative to a total mass of the coating agent, of a hydroxypropyl cellulose having hydroxypropyl groups in an amount of more than 50% to 60% by mass, relative to a total mass of the hydroxypropyl cellulose, wherein the hydroxypropyl cellulose has a viscosity of 3.0 mPa•s to 5.9 mPa•s, when the hydroxypropyl cellulose is made to be 2% by mass of an aqueous solution at 20°C; and a coating ratio is 4% to 5% by mass, the coating ratio being determined by the following formula:

$$\text{Coating ratio (\% by mass)} = \frac{\text{Mass of solid preparation after coating - Mass of plain tablet}}{\text{Mass of plain tablet} + \text{Mass of solid content of coating agent used for coating}} \times 100$$

## Patentansprüche

1. Feste Formulierung, die Folgendes umfasst: eine glatte Tablette; und eine Beschichtung auf der glatten Tablette, wobei die Beschichtung eine Dicke von 45 $\mu$m bis 65 $\mu$m aufweist und mittels eines Beschichtungsmittels gebildet ist, das, bezogen auf die Gesamtmasse des Beschichtungsmittels, 3 Masse-% bis 6 Masse-% einer Hydroxypropylcellulose umfasst, die Hydroxypropylgruppen in einer Menge von mehr als 50 Masse-% bis 60 Masse-%, bezogen auf die Gesamtmasse der Hydroxypropylcellulose, umfasst, wobei die Hydroxypropylcellulose eine Viskosität von 3,0 mPa·s bis 5,9 mPa·s aufweist, wenn die Hydroxypropylcellulose so hergestellt ist, dass sie 2 Masse-% einer wässrigen Lösung bei 20 °C ausmacht; und wobei das Beschichtungsverhältnis 4 Masse-% bis 5 Masse-% beträgt, wobei das Beschichtungsverhältnis durch folgende Formel bestimmt ist:

$$\text{Beschichtungsverhältnis (Masse-\%)} = \frac{\text{Masse des festen Präparats nach Beschichtung - Masse der glatten Tablette}}{\text{Masse der glatten Tablette} + \text{Masse des Feststoffanteils des verwendeten Beschichtungsmittels}} \times 100$$

## Revendications

1. Formulation solide comprenant : un comprimé lisse ; et un enrobage sur le comprimé lisse, dans laquelle l'enrobage a une épaisseur de 45 $\mu$m à 65 $\mu$m et est formé avec un agent d'enrobage comprenant 3 % à 6 % en masse, par rapport à la masse totale de l'agent d'enrobage, d'une hydroxypropylcellulose ayant des groupes hydroxypropyle en une quantité de plus de 50 % à 60 % en masse, par rapport à la masse totale de l'hydroxypropylcellulose, et dans laquelle l'hydroxypropylcellulose a une viscosité de 3,0 mPa.s à 5,9 mPa.s quand l'hydroxypropylcellulose est mise sous la forme d'une solution aqueuse à 2 % en masse à 20°C ; et le rapport d'enrobage est de 4 % à 5 % en masse, le rapport d'enrobage étant déterminé par la formule suivante :

$$\text{Rapport d'enrobage (\% en masse)} = \frac{\text{Masse de la préparation solide après enrobage} - \text{Masse du comprimé lisse}}{\text{Masse du comprimé lisse} + \text{Masse de la teneur en solides de l'agent d'enrobage utilisé pour l'enrobage}} \times 100$$

## FIG. 1

## FIG. 2

FIG. 3

FIG. 4

x50

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2013126671 A
- JP 2001031701 A
- JP HEI9202801 B
- JP 2007001873 A
- WO 2011027728 A
- JP 2002207030 A

- US 20060177509 A1
- US 4258179 A
- EP 1967183 A1
- EP 1588708 A1
- US 20120157674 A1